(19) Europäisches Patentamt
European Patent Office
Office européen des brevets

(11) EP 2 821 394 A1

(12) **EUROPEAN PATENT APPLICATION**

(43) Date of publication:
**07.01.2015 Bulletin 2015/02**

(21) Application number: **13382258.5**

(22) Date of filing: **01.07.2013**

(51) Int Cl.:
***C07C 291/10*** *(2006.01)*
***A61P 1/00*** *(2006.01)*
***A61K 31/277*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fundacion MEDINA.**
**Centro de Excelencia en Investigacion de Medicamentos Innovadores en Andalucia**
**18016 Armilla - Granada (ES)**

(72) Inventors:
- **Genilloud Rodriguez, Olga**
  **E-28006 Madrid (ES)**
- **Tormo Beltran, José Rubén**
  **E-28002 MADRID (ES)**
- **Reyes Benitez, José Fernando**
  **E-18150 Gójar - Granada (ES)**
- **El Aouad, Noureddine**
  **E-18007 Granada (MA)**
- **Vicente Perez, Maria Francisca**
  **E-28020 Madrid (ES)**
- **De La Cruz Moreno, Mercedes**
  **E-18008 Granada (ES)**
- **Bills, Gerald Fremont**
  **Pearland, TX Texas 77584 (US)**
- **Gonzalez Menendez, Victor Manuel**
  **E-18007 Granada (ES)**
- **Monteiro De Aguiar, Maria Cândida**
  **28002 Madrid (ES)**

(74) Representative: **ABG Patentes, S.L.**
**Avenida de Burgos, 16D**
**Edificio Euromor**
**28036 Madrid (ES)**

(54) **Compounds with antibacterial activity**

(57) The present invention relates to compounds of general formula (I)

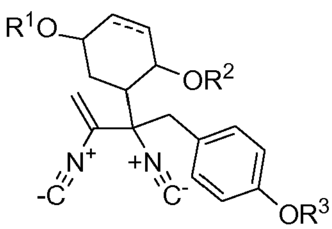

(I)

wherein $R^1$, $R^2$, $R^3$ and - - - - - take various meanings, pharmaceutical compositions containing them and their use in medicine, particularly for the treatment and/or prophylaxis of a bacterial infection disease.

EP 2 821 394 A1

## Description

### FIELD OF THE INVENTION

The present invention relates to new compounds, pharmaceutical compositions containing them and their use in medicine, particularly as agents able to treat, prevent or control bacterial infections. The present invention also relates to processes for preparing such compounds and compositions.

### BACKGROUND OF THE INVENTION

Multitude of substances exhibiting antibacterial activity are known in the State of the Art. Some examples of known antibacterial drugs include quinolones (fluoroquinolones, nalidixic acid), cephalosporins (amoxicillin, cephamycin, ceftazidime), penicillins (aminopinicillins, carboxypenicillin, mecillinam), carbapenems (imipenem, ertapenem, meropenem), macrolides (erythromycin, azythromycin), telithromycin, clindamycin, fidaxomicin), glycopeptides (vancomycin, teicoplanin), fosfomycins, aminoglycosides (gentamicin, amikacin, kanamycin), tetracyclines (tigecycline, doxycycline), oxazolidinones (linezolid), rifampicin, chloramphenicol, lipopeptides (polymyxins, colistin), cyclic lipopeptides (daptomycin), sulfonamides (trimethoprim/sulfamethoxazole), etc...

However, infections caused by bacteria are a growing medical concern as many of these pathogens are resistant to various common antibiotics. Such microbes include *Staphylococcus aureus, Staphylococcus epidermidis, Staphylococcus hemolyticus, Streptococcus pyogenes, Streptococcus pneumoniae, Enterococcus faecalis, Enterococcus faecium, Pseudomonas aeruginosa, Actinobacter baumannii, Escherichia coli, Stenotrophomonas maltophilia, Clostridium difficile* and other pathogenic bacteria. See: F. D. Lowy The Journal of Clinical Investigation 2003, 111 (9), 1265; George Talbot et al., Bad Bugs Need Drugs: An Update on the Development Pipeline from the Antimicrobial Availability Task Force of the Infectious Disease Society of America, 42 CLINICAL INFECTIOUS DISEASES 657 (2006); Brad Spellberg et al., The Epidemic of Antibiotic-Resistant Infections: A Call to Action for the Medical Community from the Infectious Disease Society of America, 46 CLINICAL INFECTIOUS DISEASES 155 (2007).

In spite of the need for new antibacterial compounds, effective against such multi-drug resistant organisms and the intense efforts applied to this field, very few new antibiotic compounds have been approved by the regulatory agencies. In this respect, the Infectious Diseases Society of America has reported in 2013 that only a single new antibiotic (ceftaroline fosamil) has been approved by the US Food and Drug Administration (FDA) since 2010, and few new drugs are in the pipeline. The report also found that only 7 new antibiotics targeting multidrug-resistant gram-negative bacilli have reached phase 2 or phase 3 trials since 2010, with the goal of developing 10 new antibiotics by 2020. See: Helen W. Boucher et al., 10 x '20 Progress-Development of New Drugs Active Against Gram-Negative Bacilli: An Update From the Infectious Diseases Society of America, Clin Infect Dis (2013).

Thus, there remains a need for potent antibiotic agents that inhibit the growth of bacteria including bacteria that are resistant to known antibiotics.

### BRIEF DESCRIPTION OF THE INVENTION

The present invention solves the aforementioned need by the provision of new compounds able to inhibit the growth of bacteria including certain bacteria that are resistant to known antibiotics.

In one aspect, the present invention is directed to compounds of general formula (I) or pharmaceutically acceptable salts, isomers, prodrugs or solvates thereof

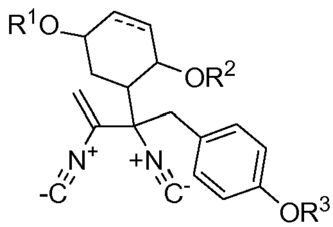

wherein

each $R^1$, $R^2$ and $R^3$ are independently selected from hydrogen and a hydroxyl protecting group; and ----- represents a single or double bond.

Another aspect of this invention refers to a medicament or pharmaceutical composition comprising at least one

compound of formula (I) as defined above, or a pharmaceutically acceptable salt, isomer, prodrug or solvate thereof and a pharmaceutically acceptable excipient.

Another aspect of this invention refers to a compound of formula (I) as defined above, or a pharmaceutically acceptable salt, isomer, prodrug or solvate thereof for use in medicine, particularly for the treatment and/or prophylaxis of a bacterial infection disease.

Another aspect of this invention refers to the use of a compound of formula (I) as defined above, or a pharmaceutically acceptable salt, isomer, prodrug or solvate thereof in the manufacture of a medicament for the treatment and/or prophylaxis of a bacterial infection disease.

Another aspect of the present invention refers to a method for the treatment and/or prophylaxis of a bacterial infection disease, the method comprising administering to the subject in need of such a treatment or prophylaxis an effective amount of a compound of formula (I) as defined above, or a pharmaceutically acceptable salt, isomer, prodrug or solvate thereof.

Thus, the present invention establishes that the compounds of general formula (I) or pharmaceutically acceptable salts, isomers, prodrugs or solvates thereof inhibit the growth of bacterial pathogens. The compounds of the invention have been found to exhibit a wide antibacterial spectrum against resistant bacteria, and specifically a strong antibacterial activity against Gram-negative bacteria. Accordingly, the compounds of the invention can be advantageously used as antibiotics.

The present invention also relates to the obtention of compounds of formula (I) from microorganisms capable of producing them, specifically from a fungus identified as *Ophiosphaerella korrae,* and more specifically from the strain *O. korrae* CBS 102216, and the formation of derivatives from the obtained compounds. In a preferred embodiment, the process for obtaining compounds of formula (I) comprises the steps of cultivating a strain of a microorganism capable of producing them such as *O. korrae* CBS 102216 in an aqueous nutrient medium with assimilable carbon and nitrogen sources and salts, under controlled submerged aerobic conditions, and then recovering and purifying the compounds according to the invention from the cultured broth.

These aspects and preferred embodiments thereof are additionally also defined hereinafter in the detailed description, as well as in the claims.

## BRIEF DESCRIPTION OF THE FIGURES

**Figure 1.** Determination of Inhibition of Macromolecular Synthesis of MDN-0057 showing signal for Cell Wall Biosynthesis at 2X MIC against strain *E. coli acrAB*::Tn903.

## DETAILED DESCRIPTION OF THE INVENTION

The number of new antibiotics to be approved by the FDA each year has fallen steadily since 1980, a trend that holds for all drugs. This has left few treatment options for certain infections, specially those caused by gram-negative bacteria, which are occasionally resistant to all the antibiotics now on the market.

The present invention relates to a family of novel natural products that possess antibacterial activity. These compounds are potent antibiotic agents with broad spectra of activity and can be used against pathogens associated with human and animal bacterial infections. Further, the antibiotic agents of this invention represent an important contribution to therapy for treating infections that are resistant to various known antibiotics.

Thus, the present invention relates to novel antibacterial compounds of general formula (I) as defined above, which exhibit antibiotic activity and have been found particularly useful against gram-negative bacteria such as *Acinetobacter baumannii, Escherichia coli, Pseudomonas aeruginosa* and *Klebsiella pneumoniae,* microbes which cause great problems at medical treatment sites as the cause of opportunistic and hospital-acquired infections.

In these compounds the groups can be selected in accordance with the following guidance:

Alkyl groups may be branched or unbranched, and preferably have from 1 to about 18 carbon atoms, such as from 1 to about 12 carbon atoms. One more preferred class of alkyl groups has from 1 to about 6 carbon atoms; even more preferred are alkyl groups having 1, 2, 3 or 4 carbon atoms. Methyl, ethyl, n-propyl, *iso*-propyl and butyl, including n-butyl, tert-butyl, sec-butyl and *iso*-butyl are particularly preferred alkyl groups in the compounds of the present invention. As used herein, the term alkyl, unless otherwise stated, refers to both cyclic and non-cyclic groups, although cyclic groups will comprise at least three carbon ring members.

Alkenyl and alkynyl groups in the compounds of the present invention may be branched or unbranched, have one or more unsaturated linkages and from 2 to about 18 carbon atoms, such as from 2 to about 12 carbon atoms. One more preferred class of alkenyl and alkynyl groups has from 2 to about 6 carbon atoms; even more preferred are alkenyl and alkynyl groups having 2, 3 or 4 carbon atoms. The terms alkenyl and alkynyl as used herein refer to both cyclic and

noncyclic groups, although cyclic groups will comprise at least three carbon ring members.

Suitable aryl groups in the compounds of the present invention include single and multiple ring compounds, including multiple ring compounds that contain separate and/or fused aryl groups. Typical aryl groups contain from 1 to 3 separated and/or fused rings and from 6 to about 18 carbon ring atoms. Preferably aryl groups contain from 6 to about 10 carbon ring atoms. Specially preferred aryl groups include substituted or unsubstituted phenyl, substituted or unsubstituted naphthyl, substituted or unsubstituted biphenyl, substituted or unsubstituted phenanthryl and substituted or unsubstituted anthryl.

The groups above mentioned may be substituted at one or more available positions by one or more suitable groups such as OR', =O, SR', SOR', $SO_2R'$, $OSO_2R'$, $OSO_3R'$, $NO_2$, NHR', $N(R')_2$, =N-R', N(R')COR', $N(COR')_2$, $N(R')SO_2R'$, N(R')C(=NR')N(R')R', CN, halogen, COR', COOR', OCOR', OCOOR', OCONHR', $OCON(R')_2$, CONHR', $CON(R')_2$, CON(R')OR', $CON(R')SO_2R'$, $PO(OR')_2$, PO(OR')R', PO(OR')(N(R')R'), substituted or unsubstituted $C_1$-$C_{12}$ alkyl, substituted or unsubstituted $C_2$-$C_{12}$ alkenyl, substituted or unsubstituted $C_2$-$C_{12}$ alkynyl, and substituted or unsubstituted aryl, wherein each of the R' groups is independently selected from the group consisting of hydrogen, OH, $NO_2$, $NH_2$, SH, CN, halogen, COH, COalkyl, COOH, substituted or unsubstituted $C_1$-$C_{12}$ alkyl, substituted or unsubstituted $C_2$-$C_{12}$ alkenyl, substituted or unsubstituted $C_2$-$C_{12}$ alkynyl, and substituted or unsubstituted aryl. Where such groups are themselves substituted, the substituents may be chosen from the foregoing list.

Suitable halogen groups or substituents in the compounds of the present invention include F, Cl, Br, and I.

Suitable protecting groups are well known for the skilled person in the art. A general review of protecting groups in organic chemistry is provided by Wuts, PGM and Greene TW in Protecting Groups in Organic Synthesis, 4th Ed. Wiley-Interscience, and by Kocienski PJ in Protecting Groups, 3rd Ed. Georg Thieme Verlag. These references provide sections on protecting groups for hydroxy and amino groups. All these references are incorporated by reference in their entirety.

Examples of such protected OH include ethers, silyl ethers, esters, sulfonates, sulfenates and sulfinates, carbonates and carbamates. In the case of ethers the protecting group for the OH can be selected from methyl, methoxymethyl, methylthiomethyl, (phenyldimethylsilyl)methoxymethyl, benzyloxymethyl, *p*-methoxybenzyloxymethyl, [(3,4-dimethoxybenzyl)oxy]methyl, *p*-nitrobenzyloxymethyl, *O*-nitrobenzyloxymethyl, [(R)-1-(2-nitrophenyl)ethoxy]methyl, (4-methoxyphenoxy)methyl, guaiacolmethyl, [(*p*-phenylphenyl)oxy]methyl, *t*-butoxymethyl, 4-pentenyloxymethyl, siloxymethyl, 2-methoxyethoxymethyl, 2-cyanoethoxymethyl, bis(2-chloroethoxy)methyl, 2,2,2-trichloroethoxymethyl, 2-(trimethylsilyl)ethoxymethyl, menthoxymethyl, *o*-bis(2-acetoxyethoxy)methyl, tetrahydropyranyl, fluorous tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, 4-methoxytetrahydrothiopyranyl *S,S*-dioxide, 1-[(2-chloro-4-methyl)phenyl]-4-methoxypiperidin-4-yl, 1-(2-fluorophenyl)-4-methoxypiperidin-4-yl, 1-(4-chlorophenyl)-4-methoxypiperidin-4-yl, 1,4-dioxan-2-yl, tetrahydrofuranyl, tetrahydrothiofuranyl, 2,3,3a,4,5,6,7,7*a*-octahydro-7,8,8-trimethyl-4,7-methanobenzofuran-2-yl, 1-ethoxyethyl, 1-(2-chloroethoxy)ethyl, 2-hydroxyethyl, 2-bromoethyl, 1-[2-(trimethylsilyl)ethoxy]ethyl, 1-methyl-1-methoxyethyl, 1-methyl-1-benzyloxyethyl, 1-methyl-1-benzyloxy-2-fluoroethyl, 1-methyl-1-phenoxyethyl, 2,2,2-trichloroethyl, 1,1-dianisyl-2,2,2-trichloroethyl, 1,1,1,3,3,3-hexafluoro-2-phenylisopropyl, 1-(2-cyanoethoxy)ethyl, 2-trimethylsilylethyl, 2-(benzylthio)ethyl, 2-phenylselenyl)ethyl, *t*-butyl, cyclohexyl, 1-methyl-1'-cyclopropylmethyl, allyl, prenyl, cinnamyl, 2-phenallyl, propargyl, *p*-chlorophenyl, *p*-methoxyphenyl, *p*-nitrophenyl, 2,4-dinitrophenyl, 2,3,5,6-tetrafluoro-4-(trifluoromethyl)phenyl, benzyl, *p*-methoxybenzyl, 3,4-dimethoxybenzyl, 2,6-dimethoxybenzyl, *o*-nitrobenzyl, *p*-nitrobenzyl, pentadienylnitrobenzyl, pentadienylnitropiperonyl, halobenzyl, 2,6-dichlorobenzyl, 2,4-dichlorobenzyl, 2,6-difluorobenzyl, *p*-cyanobenzyl, fluorous benzyl, 4-fluorousalkoxybenzyl, trimethylsilylxylyl, *p*-phenylbenzyl, 2-phenyl-2-propyl, *p*-acylaminobenzyl, *p*-azidobenzyl, 4-azido-3-chlorobenzyl, 2-trifluoromethylbenzyl, 4-trifluoromethylbenzyl, *p*-(methylsulfinyl)benzyl, *p*-siletanylbenzyl, 4-acetoxybenzyl, 4-(2-trimethylsilyl)ethoxymethoxybenzyl, 2-naphthylmethyl, 2-picolyl, 4-picolyl, 3-methyl-2-picolyl *N*-oxido, 2-quinolinylmethyl, 6-methoxy-2-(4-methylphenyl-4-quinolinemethyl, 1-pyrenylmethyl, diphenylmethyl, 4-methoxydiphenylmethyl, 4-phenyldiphenylmethyl, *p,p*'-dinitrobenzhydryl, 5-dibenzosuberyl, triphenylmethyl, tris(4-*t*-butylphenyl)methyl, $\alpha$-naphthyldiphenylmethyl, *p*-methoxyphenyldiphenylmethyl, di(*p*-methoxyphenyl)phenylmethyl, tri(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 4,4'-dimethoxy-3"-[*N*-(imidazolylmethyl)]trityl, 4,4'-dimethoxy-3"-[*N*-(imidazolylethyl)carbamoyl]trityl, bis(4-methoxyphenyl)-1'-pyrenylmethyl, 4-(17-tetrabenzo[*a,c,g,i*]fluorenylmethyl-4,4"-dimethoxytrityl, 9-anthryl, 9-(9-phenyl)xanthenyl, 9-phenylthioxanthyl, 9-(9-phenyl-10-oxo)anthryl, 1,3-benzodithiolan-2-yl, and 4,5-bis(ethoxycarbonyly[1,3]-dioxolan-2-yl, benzisothiazolyl *S,S*-dioxido. In the case of silyl ethers the protecting group for the OH can be selected from trimethylsilyl, triethylsilyl, triisopropylsilyl, dimethylisopropylsilyl, diethylisopropylsilyl, dimethylhexylsylil, 2-norbomyldimethylsilyl, *t*-butyldimethylsilyl, *t*-butyldiphenylsilyl, tribenzylsilyl, tri-*p*-xylylsilyl, triphenylsilyl, diphenylmethylsilyl, di-*t*-butylmethylsilyl, bis(*t*-butyl)-1-pyrenylmethoxysilyl, tris(trimethylsilyl)silyl, (2-hydroxystyryl)dimethylsilyl, (2-hydroxystyryl)diisopropylsilyl, *t*-butylmethoxyphenylsilyl, *t*-butoxydiphenylsilyl, 1,1,3,3-tetraisopropyl-3-[2-(triphenylmethoxy)ethoxy]disiloxane-1-yl, and fluorous silyl. In the case of esters the protecting group for the OH can be selected from formate, benzoylformate, acetate, chloroacetate, dichloroacetate, trichloroacetate, trichloroacetamidate, trifluoroacetate, methoxyacetate, triphenylmethoxyacetate, phenoxyacetate, *p*-chlorophenoxyacetate, phenylacetate, diphenylacetate, 3-phenylpropionate, bisfluorous chain type propanoyl, 4-pentenoate, 4-oxopentanoate, 4,4-(ethylenedithio)pentanoate, 5[3-bis(4-methoxyphenyl)hydroxymethylphenoxy]levulinate, pivaloate, 1-adamantoate, crotonate, 4-methoxycrotonate, benzoate, *p*-phenylbenzoate, 2,4,6-trimethylbenzoate, 4-bromobenzoate, 2,5-difluorobenzoate, p-nitrobenzoate, picolinate, nicotinate, 2-(azidomethyl)benzoate, 4-azidobutyrate, (2-azidomethyl)phenylacetate, 2-{[(tritylthio)oxy]methyl}benzoate, 2-{[(4-methoxytritylthio)oxy]methyl}benzoate, 2-{[methyl(tritylthio)amino]methyl}benzoate, 2-{{[(4-methoxytrityl)thio]methylamino}-methyl}benzoate, 2-(allyloxy)phenylacetate, 2-(prenyloxymethyl)benzoate, 6-(levulinyloxymethyly3-methoxy-2-nitrobenzoate, 6-(levulinyloxymethyl)-3-methoxy-4-nitrobenzoate, 4-benzyloxybutyrate, 4-trialkylsilyloxybutyrate, 4-acetoxy-2,2-dimethylbutyrate, 2,2-dimethyl-4-pentenoate, 2-iodobenzoate, 4-nitro-4-methylpentanoate, *o*-(dibromomethyl)benzoate, 2-formylbenzenesulfonate, 4-(methylthiomethoxy)butyrate, 2-(methylthiomethoxymethyl)benzoate, 2-(chloroacetoxymethyl)benzoate, 2-[(2-chloroacetoxy)ethyl]benzoate, 2-[2-(benzyloxy)ethyl]benzoate, 2-[2-(4-methoxybenzyloxy)ethyl]benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o*-(methoxycarbonyl)benzoate, α-naphthoate, nitrate, alkyl *N,N,N,N*-tetramethylphosphorodiamidate, and 2-chlorobenzoate. In the case of sulfonates, sulfenates and sulfinates the protecting group for the OH can be selected from sulfate, allylsulfonate, methanesulfonate, benzylsulfonate, tosylate, 2-[(4-nitrophenyl)ethyl]sulfonate, 2-trifluoromethylbenzenesulfonate, 4-monomethoxytritylsulfenate, alkyl 2,4-dinitrophenylsulfenate, 2,2,5,5-tetramethylpyrrolidin-3-one-1-sulfinate, borate, and dimethylphosphinothiolyl. In the case of carbonates the protecting group for the OH can be selected from methyl carbonate, methoxymethyl carbonate, 9-fluorenylmethyl carbonate, ethyl carbonate, bromoethyl carbonate, 2-(methylthiomethoxy)ethyl carbonate, 2,2,2-trichloroethyl carbonate, 1,1-dimethyl-2,2,2-trichloroethyl carbonate, 2-(trimethylsilyl)ethyl carbonate, 2-[dimethyl(2-naphthylmethyl)silyl]ethyl carbonate, 2-(phenylsulfonyl) ethyl carbonate, 2-(triphenylphosphonio)ethyl carbonate, cis-[4-[[(methoxytrityl)sulfenyl]oxy]tetrahydrofuran-3-yl]oxy carbonate, isobutyl carbonate, *t*-butyl carbonate, vinyl carbonate, allyl carbonate, cinnamyl carbonate, propargyl carbonate, p-chlorophenyl carbonate, *p*-nitrophenyl carbonate, 4-ethoxy-1-naphthyl carbonate, 6-bromo-7-hydroxycoumarin-4-ylmethyl carbonate, benzyl carbonate, *o*-nitrobenzyl carbonate, *p*-nitrobenzyl carbonate, *p*-methoxybenzyl carbonate, 3,4-dimethoxybenzyl carbonate, anthraquinon-2-ylmethyl carbonate, 2-dansylethyl carbonate, 2-(4-nitrophenyl)ethyl carbonate, 2-(2,4-dinitrophenyl)ethyl carbonate, 2-(2-nitrophenyl)propyl carbonate, alkyl 2-(3,4-methylenedioxy-6-nitrophenyl)propyl carbonate, 2-cyano-1-phenylethyl carbonate, 2-(2-pyridyl)amino-1-phenylethyl carbonate, 2-[*N*-methyl-*N*-(2-pyridyl)]amino-1-phenylethyl carbonate, phenacyl carbonate, 3',5'-dimethoxybenzoin carbonate, methyl dithiocarbonate, and *S*-benzyl thiocarbonate. And in the case of carbamates the protecting group for the OH can be selected from dimethylthiocarbamate, *N*-phenylcarbamate, *N*-methyl-*N*-(*o*-nitrophenyl)carbamate. The mention of these groups should be not interpreted as a limitation of the scope of the invention, since they have been mentioned as a mere illustration of protecting groups for OH, but further groups having said function may be known by the skilled person in the art, and they are to be understood to be also encompassed by the present invention.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions. The definition includes in particular physiologically acceptable salts; this term must be understood as equivalent to "pharmacologically acceptable salts" or "pharmaceutically acceptable salts".

The term "physiologically acceptable salt" or "pharmaceutically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid, picric acid and/or aspartic acid. Examples of physiologically tolerated salts of particular bases are salts of alkali metals and alkaline earth metals and with $NH_4$.

For those persons skilled in the art, it will be evident that the scope of the present invention also includes salts which are not pharmaceutically acceptable as possible means for obtaining pharmaceutically acceptable salts.

The term "solvate" according to this invention is to be understood as meaning any form of the compound according to the invention in which this compound has attached to it via non-covalent binding another molecule (most likely a polar solvent) especially including hydrates and alcoholates, e.g. methanolate. Methods of solvation are generally known within the art. The compounds of the invention may present different polymorphic forms, and it is intended that the invention encompasses all such forms.

Any compound that is a prodrug of a compound of formula (I) is also within the scope of the invention. The term "prodrug" is used in its broadest sense and encompasses those derivatives that are converted *in vivo* to the compounds of the invention. Examples of prodrugs include, but are not limited to derivatives of the compounds of formula

(I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley), "Design and Applications of Prodrugs" (H. Bundgaard ed., 1985, Harwood Academic Publishers) and Krogsgaard-Larsen et al. "Textbook of Drug design and Discovery" Taylor & Francis (April 2002).

Any compound referred to herein is intended to represent such specific compound as well as certain variations or forms. The compounds of the present invention represented by the above described formula (I) include isomers such as stereoisomers. The term "stereoisomer" as used herein includes any enantiomer, diastereomer or geometric isomer (E/Z) of such compound. In particular, compounds referred to herein may have asymmetric centres and therefore exist in different enantiomeric or diastereomeric forms. Thus any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same or different than the stereoisomerism of the other double bonds of the molecule. All the stereoisomers including enantiomers, diastereoisomers and geometric isomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are enamine-imine, amide-imidic acid, keto-enol, lactam-lactim, etc.

Unless otherwise stated, the compounds of the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by $^{13}C$- or $^{14}C$-enriched carbon, or the replacement of at least one nitrogen by $^{15}N$-enriched nitrogen are within the scope of this invention.

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

To provide a more concise description, some of the quantitative expressions given herein are not qualified with the term "about". It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

Preferred compounds of formula (I) are those having the absolute configuration depicted in formula (I') below

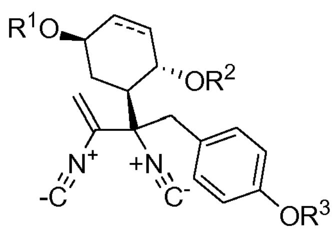

wherein

$R^1$, $R^2$, $R^3$ and ----- are as defined above in general formula (I).

According to a particular embodiment, in the compounds of general formula (I) and (I') preferably each $R^1$, $R^2$ and $R^3$ are independently H or a hydroxyl protecting group selected from:

- ethers of formula -R';

- esters of formula -C(=O)R'; and

- carbonates of formula -C(=O)OR';

wherein R' can be independently selected from substituted or unsubstituted alkyl and substituted or unsubstituted aryl.

As the skilled person knows, hydroxyl protecting groups are commonly named considering the oxygen atom. Thus, terms such as "ether", "ester" and "carbonate" really refer herein to the chemical group formed by $R^1$-$R^3$ with the oxygen atom i.e. - OR', -OC(=O)R' or -OC(=O)OR', respectively.

More particularly, preferred hydroxy protecting groups include ethers such as methyl ether, *tert-butyl* ether, benzyl ether, p-methoxybenzyl ether, 3,4-dimethoxybenzyl ether, trityl ether, allyl ether, methoxymethyl ether, 2-methoxyethoxymethyl ether, benzyloxymethyl ether, p-methoxybenzyloxymethyl ether, 2-(trimethylsilyl)ethoxymethyl, 1-methoxyethyl ether, 1-ethoxyethyl ether, 1-n-propoxyethyl ether, 1-isopropoxyethyl ether, 1-*n*-butoxyethyl ether, 1-isobutoxyethyl ether, 1-sec-butoxyethyl ether, 1-*tert*-butoxyethyl ether, 1-ethoxy-n-propyl ether, methoxypropyl ether, ethoxypropyl ether, 1-methoxy-1-methylethyl ether, 1-ethoxy-1-methylethyl ether; tetrahydropyranyl and related ethers; esters such as acetate (which is substituted in some embodiments; for instance, hydroxyacetate), benzoate, pivaloate, methoxyacetate, chloroacetate, levulinate; carbonates such as benzyl carbonate, p-nitrobenzyl carbonate, tert-butyl carbonate, 2,2,2-trichloroethyl carbonate, 2-(trimethylsilyl)ethyl carbonate, allyl carbonate.

According to a preferred embodiment, in the compounds of general formula (I) and (I') $R^1$ is H.

According to a preferred embodiment, in the compounds of general formula (I) and (I') $R^2$ is H, $-COCH_3$ or $-COCH_2OH$, more preferably $R^2$ is $-COCH_3$ or $-COCH_2OH$.

According to a preferred embodiment, in the compounds of general formula (I) and (I') $R^3$ is H or $C_1$-$C_{18}$ alkyl, more preferably $R^3$ is $C_1$-$C_4$ alkyl and still more preferably $R^3$ is methyl.

Particularly preferred compounds of the invention are the following:

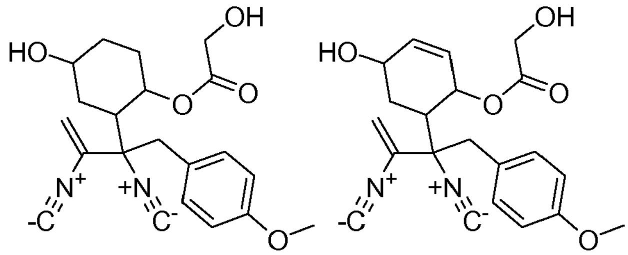

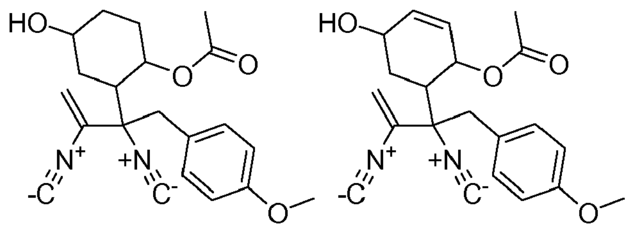

Even more preferred particular compounds of the invention are the following, referred herein to as MDN-0057, MDN-0058, MDN-0059 an MDN-0060:

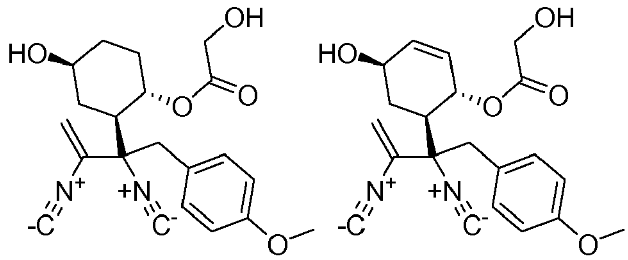

MDN-0057 MDN-0058

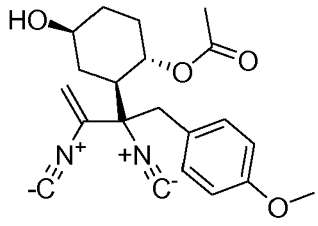

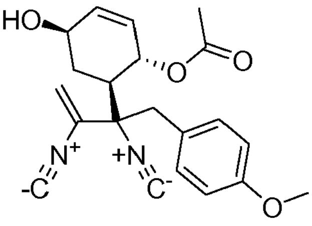

**MDN-0059** **MDN-0060**

or a pharmaceutically acceptable salt, prodrug or solvate thereof.

In additional preferred embodiments, the preferences described above for the different groups and substituents in the formulae above are combined. The present invention is also directed to such combinations.

The compounds MDN-0057, MDN-0058, MDN-0059 and MDN-0060 were isolated from the fungus identified as *Ophiosphaerella korrae,* an endophytic fungus isolated from *Cynodon dactylon* collected in USA. The fungus is deposited in the culture collection of Fundación MEDINA under the accession number CF-244,514 and has been also deposited under the Budapest Treaty in culture collection of the Centraalbureau voor Schimmelcultures, Uppsalalaan 83584 CT Utrecht, the Netherlands with accession number CBS 102216.

In the present invention, a novel family of natural products isolated from the fungal fermentation of *Ophiosphaerella korrae* is described. These compounds display antibacterial activity against various pathogens, many of which have demonstrated resistance to currently available antibiotics.

The compounds MDN-0057, MDN-0058, MDN-0059 and MDN-0060 may be obtained by cultivating *O. korrae* CBS 102216 in a suitable nutrient medium, such as those described below, until a significant amount accumulates in the fermentation.

The strain is usually cultured in an aqueous nutrient medium containing sources of assimilable carbon and nitrogen. For example, the cultures may be grown under submerged aerobic conditions (e.g., shaking the culture, submerging the culture, etc.) or in solid state fermentations. The aqueous medium is preferably maintained at a pH of about 6-8, at the initiation and termination (harvest) of the fermentation process. The desired pH may be maintained by the use of a buffer, such as morpholinoethanesulfonic acid (MES), morpholinopropane-sulfonic acid (MOPS), and the like, or by choosing nutrient materials that inherently possess buffering properties. Suitable sources of carbon in the nutrient medium include carbohydrates, such as glucose, xylose, galactose, glycerine, starch, sucrose, dextrin and the like. Other suitable carbon sources that may be used include maltose, rhamnose, raffinose, arabinose, mannose, sodium succinate and the like. Suitable sources of nitrogen are yeast extracts, meat extracts, peptone, gluten meal, cottonseed meal, soybean meal and other vegetable meals (partially or totally defatted), casein hydrolysates, soybean hydrolysates, yeast hydrolysates, corn steep liquor, dried yeast, wheat germ, feather meal, peanut powder, distiller's solubles and the like, as well as inorganic and organic nitrogen compounds, such as ammonium salts (including ammonium nitrate, ammonium sulfate, ammonium phosphate, etc.), urea, amino acids, and the like. The carbon and nitrogen sources, which may be advantageously employed in combination, need not be used in their pure forms; because less pure materials, which contain traces of growth factors, vitamins and significant quantities of mineral nutrients, are also suitable for use. When appropriate, mineral salts, such as sodium or calcium carbonate, sodium or potassium phosphate, sodium or potassium chloride, sodium or potassium iodide, magnesium salts, copper salts, cobalt salts, and the like, may be added to the medium. If necessary, especially when a culture medium foams excessively, one or more defoaming agent(s), such as liquid paraffin, fatty oils, plant oils, mineral oils or silicones, may be added. Submerged aerobic cultural conditions are typical methods of culturing cells for the production of cells in massive amounts. For small-scale production, a shaken or surface culture in a flask or bottle may be employed. When growth is carried out in large tanks, it may be preferable to use the vegetative forms of the organism for inoculation in the production tanks in order to avoid growth lag in the process of production. Accordingly, it may be desirable first to produce a vegetative inoculum of the organism by inoculating a relatively small quantity of culture medium with spores or mycelia of the organism produced in a "slant" or Petri dish and culturing said inoculated medium, also called the "seed medium," and then to transfer the cultured vegetative inoculum aseptically to large tanks. The fermentation medium, in which the inoculum is produced, is generally autoclaved to sterilize the medium prior to inoculation. The pH of the medium is generally adjusted to about 6-7 prior to the autoclaving step. Agitation and aeration of the culture mixture may be accomplished in a variety of ways. Agitation may be provided by a propeller or similar mechanical agitation equipment, by revolving or shaking the fermentor, by various pumping equipment, or by the passage of sterile air through the medium. The fermentation is usually conducted at a temperature between about 20 °C and 30 °C, such as between about 22 °C and 25 °C, for a period of about 7 to 28 days, and parameters may be varied according to fermentation conditions and scales. Preferred culturing/production media for carrying out the fermentation include the media as set forth in the examples.

Separation and purification of compounds of the present invention from the crude active extract can be performed using the proper combination of conventional chromatographic techniques.

Additionally, compounds of the invention can be obtained by modifying those already obtained from the natural source or by further modifying those already modified by using a variety of chemical reactions, for instance by means of derivatization, protection/deprotection and isomerisation reactions. Thus, hydroxyl groups can be acylated by standard coupling or acylation procedures, for instance by using acetic acid, acetyl chloride or acetic anhydride in pyridine or the like. Formate groups can be obtained by heating hydroxyl precursors in formic acid. Carbamates can be obtained by heating hydroxyl precursors with isocyanates. Hydroxyl groups can be converted into halogen groups through intermediate sulfonates for iodide, bromide or chloride, or directly using a sulfur trifluoride for fluorides; or they can be reduced to hydrogen by reduction of intermediate sulfonates. Hydroxyl groups can also be converted into alkoxy groups by alkylation using an alkyl bromide, iodide or sulfonate, or into amino lower alkoxy groups by using, for instance, a protected 2-bromoethylamine. Amido groups can be alkylated or acylated by standard alkylation or acylation procedures, for instance by using, respectively, KH and methyl iodide or acetyl chloride in pyridine or the like. Ester groups can be hydrolized to carboxylic acids or reduced to aldehyde or to alcohol. Carboxylic acids can be coupled with amines to provide amides by standard coupling or acylation procedures. When necessary, appropriate protecting groups can be used on the substituents to ensure that reactive groups are not affected. The procedures and reagents needed to prepare these derivatives are known to the skilled person and can be found in general textbooks such as March's Advanced Organic Chemistry 6th Edition 2007, Wiley Interscience. As the skilled person will appreciate, certain compounds of formula (I) may be useful as intermediate products in the preparation of other compounds of formula (I).

An important feature of the above described compounds is their bioactivity and in particular their antibacterial activity against various pathogens. Thus, a further aspect of the present invention relates to a medicament or composition in different pharmaceutical forms comprising at least a compound of formula (I), optionally at least another antibacterial drug and at least one pharmaceutically acceptable excipient for use in the treatment and/or prevention of a bacterial infection disease.

Examples of pharmaceutical compositions include any solid (tablets, pills, capsules, granules, etc.) semi-solid (creams, ointments, etc.) or liquid (solutions, suspensions or emulsions) composition for oral, topical or parenteral (such as intravenous) administration.

The respective medicament may - depending on its route of administration - also contain one or more excipients known to those skilled in the art. The medicament according to the present invention may be produced according to standard procedures known to those skilled in the art.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong *in vivo* drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

As used herein, the terms "treat", "treating" and "treatment" include in general the eradication, removal, reversion, alleviation, modification, or control of a bacterial disease after its onset.

As used herein, the terms "prevention", "preventing", "preventive", "prevent" and prophylaxis refer to the capacity of a given substance, composition or medicament to avoid, minimize or difficult the onset or development of a bacterial disease before its onset.

The compounds of the invention can be used against a variety of pathogenic bacteria including both gram-positive and gram-negative bacteria. In one particular embodiment, the compounds of formula (I) are used against one bacteria selected from the group consisting of *Staphylococcus aureus, Staphylococcus epidermidis, Staphylococcus hemolyticus, Streptococcus pyogenes, Streptococcus pneumoniae, Enterococcus faecalis, Enterococcus faecium, Pseudomonas aeruginosa, Actinobacter baumannii, Escherichia coli, Stenotrophomonas maltophilia,* and *Clostridium difficile.*

Preferably, bacterial diseases which can be treated in the context of the invention include those produced by Gram negative pathogens including species primarily causing respiratory problems (*Hemophilus influenzae, Klebsiella pneumoniae, Legionella pneumophila, Pseudomonas aeruginosa*), primarily urinary problems (*Escherichia coli, Proteus*

*mirabilis, Enterobacter cloacae, Serratia marcescens),* and primarily gastrointestinal problems (*Helicobacter pylori, Salmonella enteritidis, Salmonella typhi*) as well as gram-negative bacteria associated with nosocomial infections such as *Acinetobacter baumannii,* which cause bacteremia, secondary meningitis, and ventilator-associated pneumonia in intensive-care units of hospital establishments.

The present invention also encompasses the combination of the compounds of formula (I) with other antibacterial drug. A combination of at least a compound of formula (I) and at least another antibacterial drug may be formulated for its simultaneous, separate or sequential administration. This has the implication that the combination of the two compounds may be administered:

- as a combination that is being part of the same medicament formulation, the two compounds being then administered always simultaneously.

- as a combination of two units, each with one of the substances giving rise to the possibility of simultaneous, sequential or separate administration.

In a particular embodiment, the compound of formula (I) is independently administered from the other antibacterial drug (i.e in two units) but at the same time.

In another particular embodiment, the compound of formula (I) is administered first, and then the other antibacterial drug is separately or sequentially administered.

In yet another particular embodiment, the other antibacterial drug is administered first, and then the compound of formula (I) is administered, separately or sequentially, as defined.

Examples of antibacterial drugs that can be used in the context of the invention in combination with at least one compound of formula (I) include, but are not limited to: quinolones (fluoroquinolones, nalidixic acid), cephalosporins (amoxicillin, cephamycin, ceftazidime), penicillins (aminopinicillins, carboxypenicillin, mecillinam), carbapenems (imipenem, ertapenem, meropenem), macrolides (erythromycin, azythromycin, telithromycin, clindamycin, fidaxomicin), glycopeptides (vancomycin, teicoplanin), fosfomycins, aminoglycosides (gentamicin, amikacin, kanamycin), tetracyclines (tigecycline, doxycycline), oxazolidinones (linezolid), rifampicin, chloramphenicol, lipopeptides (polymyxins, colistin), cyclic lipopeptides (daptomycin), sulfonamides (Trimethoprim/Sulfamethoxazole).

Another aspect of the invention is a method of treatment of a patient, notably a human, suffering a bacterial infection disease, or likely to suffer a bacterial disease, which comprises administering to the patient in need of such a treatment or prophylaxis an effective amount of a compound of formula (I).

By an "effective" amount or a "therapeutically effective amount" of a drug or pharmacologically active agent is meant a nontoxic but sufficient amount of the drug or agent to provide the desired effect. In the therapy of the present invention, an "effective amount" of the compound of formula (I) is the amount of that compound that is effective to provide the desired effect. The amount that is "effective" will vary from subject to subject, depending on the age and general condition of the individual, the particular active agent or agents, and the like. Thus, it is not always possible to specify an exact "effective amount". However, an appropriate "effective" amount in any individual case may be determined by one of ordinary skill in the art using routine experimentation.

The following examples are merely illustrative of certain embodiments of the invention and cannot be considered as restricting it in any way.

## EXAMPLES

### EXAMPLE 1: FERMENTATION IN ERLENMEYER FLASKS

The antibacterial activity of the agents produced by strain CBS102216 was initially detected in a 12-medium nutritional array in 1-mL microfermentations in deepwell 96-well plates (G. Bills, G. Platas, A. Fillola, M.R. Jiménez, J. Collado, F. Vicente, J. Martín, A. González, J. Bur-Zimmermann, J.R. Tormo & F. Peláez. 2008. Enhancement of antibiotic and secondary metabolite detection from filamentous fungi by growth on nutritional arrays. Journal of Applied Microbiology 104:1644-1658). The most potent activity from extracts of a medium designated YES (sucrose (Sigma) 150 g, yeast extract (Bacto) 20 g, $MgSO_4.7H_2O$ 0.5 g, trace elements solution 1mL ($ZnSO_4.7H_2O$ 1 g, $CuSO_4.5H_2O$ 0.5g to 100mL) 1000 mL distilled $H_2O$) was selected for further study.

To further characterize the molecule responsible for the activity, a 1-L fermentation was prepared. Three to four mycelial discs were used to inoculate 250 mL Erlenmeyer flasks containing 50 mL of seed medium SMYA of the following composition (in g/L): (neopeptone (Bacto) 10 g, maltose (Sigma) 40 g, yeast extract (Bacto) 10 g, agar 4 g). Flasks were incubated on an orbital shaker (220 rpm, 5 cm throw) at 22 °C, 70% relative humidity, to produce homogenous hyphal suspensions. After growing the inoculum stage for 7 days, aliquots of this seed culture were used to inoculate 1 L fermentations using YES as base medium (sucrose (Sigma) 150 g , yeast extract (Bacto) 20 g, $MgSO_4.7H_2O$ 0.5 g,

trace elements solution 1 mL ($ZnSO_4.7H_2O$ 1 g, $CuSO_4.5H_2O$ 0.5g to 100mL) in 1000 mL distilled $H_2O$). Fermentations in 500 mL flasks containing 200 mL of liquid YES were inoculated with aliquots of 3 mL seed and incubated on a gyratory shaker (220 r.p.m) during 14 days at 22°C.

EXAMPLE 2: ISOLATION AND STRUCTURAL IDENTIFICATION OF MDN-0057, MDN-0058, MDN-0059 AND MDN-0060

The fermentation broth (5 L) was extracted with acetone (5 L) under continuous shaking at 220 rpm for 2.5 h. The mycelium was separated by centrifugation and the supernatant (10 L) was concentrated to 5 L under nitrogen stream removing most of the acetone. After paper filtration, the remaining solution was loaded with continuous 1:1 water dilution onto a column packed with SP-207SS reversed phase resin (brominated styrenic polymer, 65 g) previously equilibrated with water. The column was further washed with water (5 L) and afterwards eluted at 8 mL/min using a linear gradient from 10% to 100% acetone in water for 40 min with a final 100% acetone wash step of 20 min collecting 28 fractions of 20 mL. Compounds of interest were identified to elute in the 4:1 acetone-water fractions by HPLC. These fractions were then classified into five different sets according to their abundance and presence of each of the components.

All five sets were further purified by reversed phase preparative HPLC (Agilent Zorbax SB-C8, 21.2 × 250 mm, 7 μm; 20 mL/min, UV detection at 210 nm) with a linear gradient of ACN in water from 5% to 45% over 15 min, a 25 min isocratic step of 45% ACN in water, a 45% to 100% linear gradient of ACN in water for 10 minutes and a final 10 min wash of 100% ACN, where MDN-0057 eluted at 16.3 min, MDN-0058 eluted at 17.0 min, MDN-0059 at 19.5 min and MDN-0060 at 20.5 min.

Preparative HPLC fractions containing the compounds of interest were further re-purified by reversed phase semi-preparative HPLC (Agilent Zorbax SB-C8, 9.3 × 250 mm, 5 μm; 3.6 mL/min, UV detection at 210 nm) with a linear gradient of ACN in water from 5% to 40% ACN over 10 min, a 25 min isocratic step of 40% ACN in water, a second 40% to 100% linear gradient of ACN in water for 10 minutes and a final 10 min wash of 100% ACN, where MDN-0057 eluted at 15.5 min, MDN-0058 eluted at 16.5 min, MDN-0059 at 22.5 min and MDN-0060 at 24.5 min.

Total yields of all four of the compounds obtained from the original 5 L fermentation were: MDN-0057: 95.8 mg; MDN-0058: 86.0 mg; MDN-0059: 10.8 mg and MDN-0060: 13.8 mg

**MDN-0057:** colorless oil; $[\alpha]^{25}D$ + 50.6 (c=0.1, MeOH); IR (ATR) v $cm^{-1}$: 3359, 3132, 3051, 2999, 2938, 2868, 2838, 2130, 2120, 1748, 1612, 1514, 1247, 1198, 1180, 1093, 1031, 733. HRMS m/z 385.1755 $[M+H]^+$ (calcd for $C_{21}H_{25}N_2O_5$, 385.1758); 402.2033 $[M+NH_4]^+$ (calcd for $C_{21}H_{28}N_3O_5$, 402.2024); $^1H$ and $^{13}C$ NMR data see Table 1;

**Table 1.** NMR Spectroscopic Data (500 MHz, $CD_3OD$) for MDN-0057

| Pos. | $\delta_C$, type | $\delta_H$, mult. (*J* in Hz) | COSY | HMBC (H to C) |
|---|---|---|---|---|
| 1 | 73.0, CH | 4.92, ddd (10.5, 10.5, 4.9) | $2_{ax}$, $2_{eq}$, 6 | 3, 6, 7, 17 |
| $2_{eq}$ | 30.4, $CH_2$ | 2.07, m | 1, $2_{ax}$, $3_{eq}$, $3_{ax}$ | 1, 3, 4 |
| $2_{ax}$ | | 1.54, m | 1, $2_{eq}$, $3_{eq}$, $3_{ax}$ | 1, 3 |
| $3_{eq}$ | 33.2, $CH_2$ | 2.00, m | $2_{ax}$, $2_{eq}$, $3_{ax}$, 4 | 1, 2, 4, 5 |
| $3_{ax}$ | | 1.41, m | $2_{ax}$, $2_{eq}$, $3_{eq}$, 4 | 1, 2, 4 |
| 4 | 69.3, CH | 3.77, m | $3_{eq}$, $3_{ax}$, $5_{eq}$, $5_{ax}$ | |
| $5_{eq}$ | 35.2, $CH_2$ | 2.43, m | 4, $5_{ax}$, 6 | 1, 3, 4, 6 |
| $5_{ax}$ | | 1.43, m | 4, $5_{eq}$, 6 | 1, 3, 4, 6, 7 |
| 6 | 45.4, CH | 2.63, ddd (10.5, 10.5, 4.0) | 1, $5_{eq}$, $5_{ax}$ | 1, 5 |
| 7 | 71.5, C | | | |
| 8 | 133.5, C | | | |
| 9 | 117.3, $CH_2$ | 5.33, d (1.8) | 9b | 7, 8, 10 |
| | | 5.06 br d (1.3) | 9a | 7, 8, 10 |
| 10 | 41.8, $CH_2$ | 3.25, d (14.1) | 10b, 12, 16 | 6, 7, 11, 12, 16 |
| | | 3.00, d (14.1) | 10a, 12, 16 | 6, 7, 11, 12, 16 |
| 11 | 126.5, C | | | |
| 12 | 132.4, CH | 7.20, d (8.7) | 10a, 10b, 13 | 10, 11, 13, 14, 16 |
| 13 | 114.6, CH | 6.86, d (8.7) | 12 | 11, 14, 15 |
| 14 | 160.9, C | | | |
| 15 | 114.6, CH | 6.86, d (8.7) | 16 | 11, 13, 14 |
| 16 | 132.4, CH | 7.20, d (8.7) | 10a, 10b, 15 | 10, 11, 12, 14, 15 |
| 17 | 173.0, C | | | |

(continued)

| Pos. | $\delta_C$, type | $\delta_H$, mult. (*J* in Hz) | COSY | HMBC (H to C) |
|---|---|---|---|---|
| 18 | 61.1, $CH_2$ | 4.04, d (17.3) | 18b | 17 |
| | | 3.92, d (17.3) | 18a | 17 |
| 19 | 55.6, $CH_3$ | 3.77, s, 3H | | 14 |
| 20 | 164.5, C | | | |
| 21 | 170.0, C | | | |

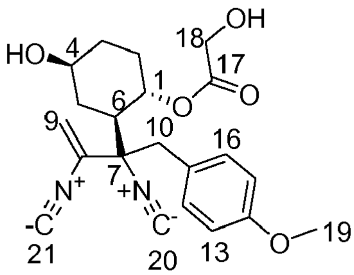

**MDN-0057**

**MDN-0058:** colorless oil; $[\alpha]^{25}_D$ + 121.0 (c=0.1, MeOH); IR (ATR) ν $cm^{-1}$: 3348, 3132, 3043, 2999, 2958, 2935, 2916, 2838,2119, 1747, 1612, 1514, 1251, 1192, 1181, 1096, 1028, 735. HRMS m/z 383.1591 $[M+H]^+$ (calcd. for $C_{21}H_{23}N_2O_5$, 383.1602); 400.1871 $[M+NH_4]^+$ (calcd. for $C_{21}H_{26}N_3O_5$, 400.1867); $^1$H and $^{13}$C NMR data see Table 2;

Table 2. NMR Spectroscopic Data (500 MHz, $CD_3OD$) for MDN-0058.

| Pos. | $\delta_C$, type | $\delta_H$, mult. (*J* in Hz) | COSY | HMBC (H to C) |
|---|---|---|---|---|
| 1 | 70.8, CH | 5.46, ddd (8.8, 3.2, 1.9) | 2, 4, 6 | 2, 3, 6, 7, 17 |
| 2 | 126.6, CH | 5.55, dt (10.1, 2.4) | 1, 3, 4 | 4, 6 |
| 3 | 137.4, CH | 6.00, dd (10.1, 1.1) | 2, 4 | 1, 5 |
| 4 | 67.1, CH | 4.47, m | 1, 2, 3, 5a, 5b | |
| 5 | 33.6, $CH_2$ | 2.54, m | 4, 5b, 6 | 1, 3, 4, 6 |
| | | 1.58, td (12.7, 10.3) | 4, 5a, 6 | 1, 4, 6 |
| 6 | 44.5, CH | 2.73, m | | 1, 4, 5, 7 |
| 7 | 71.8, C | | | |
| 8 | 134.1, C | | | |
| 9 | 118.6, $CH_2$ | 5.41, d (1.7) | 9b | 7, 8 |
| | | 5.24 br s | 9a | 7, 8 |
| 10 | 41.0, $CH_2$ | 3.35, d (14.1) | 10b | 6, 7, 11, 12, 16 |
| | | 3.12, d (14.1) | 10a | 6, 7, 11, 12, 16 |
| 11 | 126.4, C | | | |
| 12 | 132.5, CH | 7.24, d (8.6) | 13 | 10, 11, 13, 14, 16 |
| 13 | 114.7, CH | 6.87, d (8.6) | 12 | 11, 12, 14, 15 |
| 14 | 160.9, C | | | |
| 15 | 114.7, CH | 6.87, d (8.6) | 16 | 11, 13, 14, 16 |
| 16 | 132.5, CH | 7.24, d (8.6) | 15 | 10, 11, 12, 14, 15 |
| 17 | 173.5, C | | | |
| 18 | 61.0, $CH_2$ | 4.10, d (17.3) | 18b | 17 |
| | | 4.06, d (17.3) | 18a | 17 |
| 19 | 55.6, $CH_3$ | 3.77, s, 3H | | 14 |
| 20 | 164.7, C | | | |
| 21 | 170.5, C | | | |

(continued)

| Pos. | $\delta_C$, type | $\delta_H$, mult. (*J* in Hz) | COSY | HMBC (H to C) |
|---|---|---|---|---|

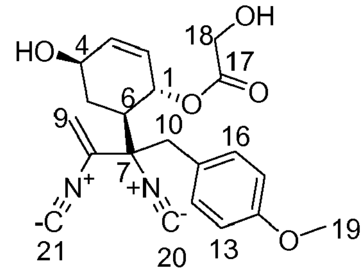

**MDN-0058**

**MDN-0059:** colorless oil; $[\alpha]^{25}_D$ + 61.0 (c=0.1, MeOH); IR (ATR) ν $cm^{-1}$: 3377, 3130, 3042, 2999, 2937, 2869, 2838, 2119, 1736, 1612, 1514, 1232, 1197, 1179, 1026, 735. HRMS m/z 369.1813 $[M+H]^+$ (calcd for $C_2$,$H_{25}N_2O_4$, 369.1809); 386.2086 $[M+NH_4]^+$ (calcd for $C_{21}H_{28}N_3O_4$, 386.2074); $^1H$ and $^{13}C$ NMR data see Table 3;

**Table 3.** NMR Spectroscopic Data (500 MHz, $CD_3OD$) for MDN-0059.

| Pos. | $\delta_C$, type | $\delta_H$, mult. (*J* in Hz) | COSY | HMBC (H to C) |
|---|---|---|---|---|
| 1 | 73.0, CH | 4.80, ddd (10.5, 10.5, 4.8) | $2_{ax}$, $2_{eq}$, 6 | 2, 3, 6, 7, 17 |
| $2_{eq}$ | 30.5, $CH_2$ | 2.04, m | 1, $2_{ax}$, $3_{eq}$, $3_{ax}$ | 1, 3, 4, 6 |
| $2_{ax}$ | | 1.50, m | 1, $2_{eq}$, $3_{eq}$, $3_{ax}$ | 3 |
| $3_{eq}$ | 33.2, $CH_2$ | 2.00, m | $2_{ax}$, $2_{eq}$, $3_{ax}$, 4 | 1, 2, 4, 5 |
| $3_{ax}$ | | 1.38, m | $2_{ax}$, $2_{eq}$, $3_{eq}$, 4 | 1, 2, 4 |
| 4 | 69.5, CH | 3.77, m | $3_{eq}$, $3_{ax}$, $5_{eq}$, $5_{ax}$ | |
| $5_{eq}$ | 35.2, $CH_2$ | 2.43, m | 4, $5_{ax}$, 6 | 1, 3, 4, 6 |
| $5_{ax}$ | | 1.45, m | 4, $5_{eq}$, 6 | 1, 3, 4, 6, 7 |
| 6 | 45.4, CH | 2.62, m | 1, $5_{eq}$, $5_{ax}$ | 1, 5 |
| 7 | 71.6, C | | | |
| 8 | 133.7, C | | | |
| 9 | 117.3, $CH_2$ | 5.35, d (1.5) | 9b | 7, 8, 10 |
| | | 5.05 br s | 9a | 7, 8, 10 |
| 10 | 41.9, $CH_2$ | 3.25, d (14.1) | 10b | 6, 7, 11, 12, 16 |
| | | 3.01, d (14.1) | 10a | 6, 7, 11, 12, 16 |
| 11 | 126.5, C | | | |
| 12 | 132.6, CH | 7.20, d (8.7) | 10a, 10b, 13 | 10, 11, 13, 14, 16 |
| 13 | 114.7, CH | 6.86, d (8.7) | 12 | 11, 14, 15 |
| 14 | 160.9, C | | | |
| 15 | 114.7, CH | 6.86, d (8.7) | 16 | 11, 13, 14 |
| 16 | 132.6, CH | 7.20, d (8.7) | 10a, 10b, 15 | 10, 12, 14, 15 |
| 17 | 171.6, C | | | |
| 18 | 21.1, $CH_3$ | 1.93, s, 3H | | 17 |
| 19 | 55.8, $CH_3$ | 3.77, s, 3H | | 14 |
| 20 | 164.3, C | | | |
| 21 | 169.8, C | | | |

(continued)

| Pos. | $\delta_C$, type | $\delta_H$, mult. (*J* in Hz) | COSY | HMBC (H to C) |
|---|---|---|---|---|

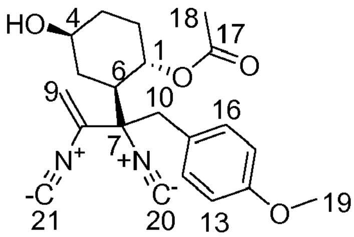

**MDN-0059**

**MDN-0060:** white amorphous solid; $[\alpha]^{25}D$ + 93.5 (c=0.1, MeOH); IR (ATR) ν $cm^{-1}$: 3457, 3130, 3040, 2998, 2935, 2876, 2836, 2118, 1735, 1612, 1514, 1229, 1023. HRMS m/z 367.1655 $[M+H]^+$ (calcd for $C_{21}H_{23}N_2O_4$, 367.1652); 384.1924 $[M+NH_4]^+$ (calcd for $C_{21}H_{26}N_3O_4$, 384.1918); $^1H$ and $^{13}C$ NMR data see Table 4;

**Table 4.** NMR Spectroscopic Data (500 MHz, $CD_3OD$) for MDN-0060.

| Pos. | $\delta_C$, type | $\delta_H$, mult. (*J* in Hz) | COSY | HMBC (H to C) |
|---|---|---|---|---|
| 1 | 70.5, CH | 5.36, ddd (8.8, 4.3, 1.9) | 2, 4, 6 | 2, 3, 6, 7, 17 |
| 2 | 126.8, CH | 5.55, dt (10.1, 2.4) | 1, 3, 4 | 4, 6 |
| 3 | 137.1, CH | 5.98, dd (10.1, 1.3) | 2, 4 | 1, 5 |
| 4 | 67.3, CH | 4.47, m | 1, 2, 3, 5a, 5b | |
| 5 | 33.7, $CH_2$ | 2.54, m | 4, 5b, 6 | 1, 3, 4, 6 |
| | | 1.58, td (12.7, 10.3) | 4, 5a, 6 | 1,3, 4, 6, 7 |
| 6 | 44.6, CH | 2.71, m | | 1, 4, 5, 7 |
| 7 | 72.0, C | | | |
| 8 | 133.1, C | | | |
| 9 | 118.5, $CH_2$ | 5.42, d (1.7) | 9b | 7, 8 |
| | | 5.22 br s | 9a | 7, 8 |
| 10 | 41.2, $CH_2$ | 3.35, d (14.1) | 10b | 6, 7, 11, 12, 16 |
| | | 3.12, d (14.1) | 10a | 6, 7, 11, 12, 16 |
| 11 | 126.6, C | | | |
| 12 | 132.6, CH | 7.24, d (8.6) | 13 | 10, 13, 14, 16 |
| 13 | 114.8, CH | 6.87, d (8.6) | 12 | 11, 14, 15 |
| 14 | 161.0, C | | | |
| 15 | 114.8, CH | 6.87, d (8.6) | 16 | 11, 13, 14 |
| 16 | 132.6, CH | 7.24, d (8.6) | 15 | 10, 12, 14, 15 |
| 17 | 172.0, C | | | |
| 18 | 20.9, $CH_3$ | 2.01, s, 3H | | 17 |
| 19 | 55.8, $CH_3$ | 3.77, s, 3H | | 14 |
| 20 | 164.6, C | | | |
| 21 | 170.4, C | | | |

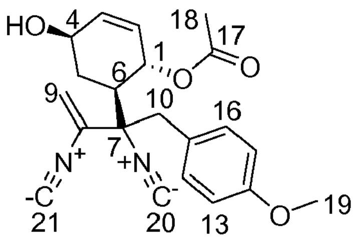

**MDN-0060**

EXAMPLE 3: DETERMINATION OF THE ABSOLUTE CONFIGURATION OF MDN-0057 TO MDN-0060

The relative configuration of the family of compounds was determined by analysis of the coupling constants measured in the $^{1}H$ NMR of MDN-0057 and NOESY correlations. The signal for proton H-1 displayed two 10.5 Hz coupling constants revealing an axial orientation of this proton in the cyclohexane ring and also confirming an axial orientation for proton H-6, corroborated by the two 10.5 Hz coupling constants measured in its signal at 2.63 ppm. The existence of a strong NOESY correlation between H-6 and H-4 revealed in turn the axial orientation of the latter proton and established the relative configuration of compound MDN-0057 as 1*S**,4*S**,6*R**. The configuration at carbon C-7 remained undetermined at this stage.

Compound MDN-0060 was selected to perform a Mosher analysis for the determination of the absolute configuration at C-4 and hence that of C-1 and C-6.

To a stirred solution of compound MDN-0060 (200 μg, 0.54 μmol) and dry pyridine (0.15 μL, 1.67 μmol, 3.1 eq.) in dry deuterochloroform (200 μL, 0.064 M) at room temperature, (R) or (S)-MTPA-Cl (1 μL, 5.4 μmol, 10 equiv.) was added. The reaction progress was monitored by analytical HPLC. After complete consumption of the compound, an aliquot of the mixture reaction was analyzed by $^{1}H$ NMR. Then the the reaction mixture was washed with water (2 × 200 μL) and the organic layer was evaporated and purified by semipreparative HPLC (Zorbax SB-C8, 9.4 × 250 mm, gradient $H_2O$-$CH_3CN$ from 5 to 100 % in 37 min then 100% $CH_3CN$ during 15 min, 3.6 mL/min, UV detection at 210 and 280 nm) to give the corresponding S-MTPA (Rt= 46.22 min, 140 μg, 44.5 %) or R-MTPA ester (Rt= 46.21 min, 160 ug, 50.8%) of MDN-0060 as white solids.

The distribution of $\delta_S$- $\delta_R$ values (Table 5) confirmed an S absolute configuration for the chiral center at C-4 and therefore the absolute configuration of the family of molecules was established as 1 S,4S,6R, remaining the configuration at the C-7 chiral center undetermined.

**Table 5.** $^{1}H$-NMR data of compounds MDN-0060 (S)-MTPA and (R)-MTPA esters and $\Delta\delta = \delta_S - \delta_R$ values

| No | MDN-0060 ($CDCl_3$) $\delta_H$, mult. (*J* in Hz) | MDN-0060 (S)-MTPA ester $\delta_H$, mult. (*J* in Hz) | MDN-0060 (R)-MTPA ester $\delta_H$, mult. (*J* in Hz) | $\Delta\delta = \delta_S - \delta_R$ (ppm) |
|---|---|---|---|---|
| 1 | 5.408, m | 5.433, m | 5.433, m | 0 |
| 2 | 5.612, dt (10.1, 2.3) | 5.714, dt (10.1, 1.9) | 5.754, dt (10.1, 2.3) | -0.040 |
| 3 | 5.994, d (10.1) | 5.846, d (10.1) | 5.969, d (10.1) | -0.123 |
| 4 | 4.529, m | 5.791, m | 5.792, m | -0.001 |
| 5 | 2.519, m | 2.749, m | 2.734, m | +0.015 |
| 5 | 1.694, m | 1.918, m | 1.838, m | +0.080 |
| 6 | 2.645, m | 2.615, m | 2.518, m | +0.097 |
| 7 | -- | -- | -- | -- |
| 8 | -- | -- | -- | -- |
| 9 | 5.290, br s | 5.296, br s | 5.291, br s | +0.005 |
| 10 | 3.276, d (14.0) | 3.246, d (14.1) | 3.205, d (13.9) | +0.041 |
| 10 | 3.040, d (14.0) | 3.071, d (14.1) | 3.051, d (13.9) | +0.020 |
| 11 | -- | -- | -- | -- |
| 12 | 7.235, d (8.6) | 7.242, d (8.4) | 7.228, d (8.5) | +0.014 |
| 13 | 6.858, d (8.6) | 6.861, d (8.4) | 6.858, d (8.5) | +0.003 |
| 14 | -- | -- | -- | -- |
| 15 | 6.858, d (8.6) | 6.861, d (8.4) | 6.858, d (8.5) | +0.003 |
| 16 | 7.235, d (8.6) | 7.242, d (8.4) | 7.228, d (8.5) | +0.014 |
| 17 | -- | -- | -- | -- |
| 18 | 2.039, s | 2.048, s | 2.051, s | -0.003 |
| 19 | 3.804, s | 3.808, s | 3.806, s | +0.002 |

(continued)

| No | MDN-0060 ($CDCl_3$) $\delta_H$, mult. (*J* in Hz) | MDN-0060 (S)-MTPA ester $\delta_H$, mult. (*J* in Hz) | MDN-0060 (R)-MTPA ester $\delta_H$, mult. (*J* in Hz) | $\Delta\delta = \delta_S - \delta_R$ (ppm) |
|---|---|---|---|---|
| 20 | -- | -- | -- | -- |
| 21 | -- | -- | -- | -- |

EXAMPLE 4: ASSESSMENT OF THE ANTIBACTERIAL ACTIVITY OF COMPOUNDS MDN-0057, MDN-0058, MDN-0059 and MDN-0060

Antibacterial activity of compounds MDN-0057, MDN-0058, MDN-0059 and MDN-0608 was evaluated using the broth microdilution methodology. This method is used to determine minimum inhibitory concentrations (MICs) which are defined as the lowest concentration of an antimicrobial compound that will inhibit the visible growth of a microorganism after overnight incubation. In such assay against a panel of bacteria selected for their resistance/susceptibility to known compounds and clinical importance, these inventive compounds are found to be effective at concentrations comparable to known antibiotics.

In the microbroth dilution assay, microorganisms were selected by streaking bacteria culture from a cryovial into Mueller Hinton II (MHII) agar plate(s) and incubate overnight at 37°C. Thereafter, 3 to 5 characteristic fresh colonies were selected and suspended in 25 mL of MHII broth media for 2 hours shaking at 220 rpm. The 2 hour broth cultures were adjusted to an optical density of 0.111 at 660 nm and then diluted to 1:100 in order to obtain assay inoculums.

The four compounds evaluated were serially diluted in 100% DMSO with a dilution factor of 2 to provide 10 compound concentrations starting at 8 mg/mL.

For the assays, 90 μL/well of the appropriated diluted inoculum were mixed with 1.6 μL/well of each compound concentration and 8.4 μL/well of MHII broth media, resulting in an additional dilution of these compounds to yield concentrations in the assay wells from 128 μg/mL to 0.25 μg/mL. After that, the assay plates were read in a Tecan Ultraevolution spectrophotometer at 612 nm for To (zero time). Then, the plates were statically incubated at 37 °C for 20 hours. After incubation, they were shaken in a DPC Micromix-5 and read again for Tf (final time). Percentage of growth inhibition was calculated using the following normalization:

$$\% \text{ Inhibition} = [1-[(T_f\text{Sample}-T_0\text{Sample})-(T_f\text{Blank}-T_0\text{blank})/(T_f\text{Growth}-T_0\text{Growth})-(T_f\text{Blank}-T_0\text{blank})] \times 100.$$

Where,

$T_{0\ Sample}$= the absorbance of the strain growth in the presence of sample measured at zero time (before incubation).

$T_{f\ Sample}$= the absorbance of the strain growth in the presence of sample measured at final time (after incubation)

$T_{0\ Growth}$= the absorbance of the strain growth in the absence of sample measured at zero time (before incubation).

$T_{f\ Growth}$= the absorbance of the strain growth in the absence of sample measured at final time (after incubation).

$T_{0\ Blank}$ = the absorbance of the broth medium (blank) measured at zero time (before incubation).

$T_{f\ Blank}$= the absorbance of the broth medium (blank) measured at final time (after incubation).

Compounds MDN-0057 and MDN-0058 showed MIC values against strains of *E. coli, P. aeruginosa, A. baumannii* and *K. pneumoniae* of 2-64 μg/mL. No activity was shown against S. *aureus* (128 μg/mL) or eukaryotic cells (>512 μg/mL). Compounds MDN-0059 and MDN-0060 showed MIC values against a wild type strain of *P. aeruginosa* of 16-64 μg/mL and against *A. baumannii* of 0.5-2 μg/mL (Table 6).

**Table 6.** Antibacterial MIC values obtained for the four tested compounds

| | | MIC (μg/mL) | | | |
|---|---|---|---|---|---|
| **STRAINS** | **DESCRIPTION** | **MDN-0057** | **MDN-0058** | **MDN-0059** | **MDN-0060** |
| *E.coli* ATCC 25922 | wild type | 16-64 | 16 | - | - |
| *E.coli*219 | ΔAcrAB::Tn903kan | 0.125-0.5 | 0.25-0.5 | - | - |
| *A.baumannii* ATCC BAA-747 | Clinical isolate | 2-4 | 1-16 | - | - |
| *A.baumannii* ATCC 19606 | Clinical isolate | 2-4 | 2-8 | - | - |
| *A.baumannii*2443 | Clinical isolate | 4 | 2 | - | - |
| *A.baumannii*2444 | ΔadeIJK | 0.25 | 0.25-1 | - | - |
| *A.baumannii*2445 | ΔadeABC | 2 | 2-8 | - | - |
| *A.baumannii*2446 | ΔadeABC, ΔadeIJK | <0.06 | <0.06 | - | - |
| *A.baumannii*5973 | Clinical isolate | 0.25 | 0.25 | 0.5-1 | 1-2 |
| *P.aeruginosa* ATCC 27853 | wild type | 2-8 | 64 | 16 | 32-64 |
| *P.aeruginosa*839 | ΔMexAB-OprM | 1-2 | 1-4 | - | - |
| *K.pneumoniae* 847 | Clinical isolate | 64 | 64 | - | - |
| *K.pneumoniae* 21 | permeable strain | 0.25 | 0.5 | - | - |
| *S.aureus* ATCC 29213 | wild type | >128 | >128 | - | - |

EXAMPLE 5: ASSESSMENT OF RESISTANCE IN RESISTANCE BIOASSAYS

The frequency of resistance to MDN-0057 and MDN-0058 was assessed on *Escherichia coli acrAB*::Tn903 (CB-219) and wild type (WT) *Acinetobacter baumannii* (ATCC BAA-747). For this purpose, the Minimum Inhibitory Concentration of MDN-0057 versus each strain was determined by broth microdilution as described in Clinical Laboratory Standards Institute publication M07-A9, Vol. 32, No. 2., "Methods for Dilution Antimicrobial Susceptibility Tests for Bacteria that Grow Aerobically; Approved Standard - Ninth Edition," January 2012. Six-well plates containing Mueller Hinton II agar (5 mL per well) were prepared with MDN-0057 or MDN-0058 at concentrations 4-fold and 8-fold above the MIC. Cultures were grown to late log phase and spread on the agar. Plates were incubated at 37 °C and examined after one and two days of incubation.

For *A. baumannii,* after 24 hours of incubation, a lawn of colonies was apparent on MDN-0057 at concentrations 4-fold and 8-fold above the broth MIC. For *E. coli acrAB*::Tn903, after 48 hours of incubation, pinpoint colonies that were too numerous to count were apparent on MDN-0057 at concentrations 4-fold and 8-fold above the broth MIC. Six to eight isolates from each species were obtained by re-streaking growth from the plates on antibiotic-free tryptic soy agar plates, and MICs of MDN-0057 versus the isolates were subsequently determined. MICs of the *E. coli* isolates were elevated at least 16-64-fold relative to their parent strain, CB-219. By contrast, MICs of the *A. baumannii* isolates were not significantly different from their parent strain, A. *baumannii* BAA-747.

**Table 7.** Micro RI bioassay on *E.coli* CB-219 for MDN-0057 and MDN-0058

| | | MIC (μg/mL) | |
|---|---|---|---|
| **STRAINS** | **DESCRIPTION** | **MDN-0057** | **MDN-0058** |
| *E.coli_219* | Parent | 0.125-0.5 | 0.25-0.5 |
| *E.coli_219,* Micro RI | Isolate #1_4X | 0.25 | 0.25 |
| *E.coli_219,* Micro RI | Isolate #2_8X | 0.25 | 0.25 |
| *E.coli_219,* Micro RI | Isolate #3_4X | 0.25 | 0.25 |
| *E.coli_219,* Micro RI | Isolate #4_4X | 0.25 | 0.25 |
| *E.coli_219,* Micro RI | Isolate #5_8X | 0.25 | 0.25 |

EXAMPLE 6. ASSESSMENT OF MODE OF ACTION USING A MACROMOLECULAR LABELING ASSAY

The macromolecular synthesis assay, in which the inhibition of the intracellular building blocks of macromolecules are measured, provides guidance for understanding the broad MOA of compounds

The inhibitory effect on synthesis of macromolecules in the lipid, cell wall, DNA, RNA and protein synthesis pathways was determined in growing cells of E. *coli acrAB*::Tn903. Cells were grown at 35°C overnight on Trypticase Soy agar, and this culture was used to inoculate 15 ml of Mueller Hinton broth II (MHB). The culture was grown to early exponential growth phase (OD600 = 0.2 to 0.3) while incubating in a shaker at 35°C and 200 rpm.

Cells in early exponential phase were exposed to MDN-0057, and their incorporation of radioactive precursors in each pathway was determined. Rifampicin, Ciprofloxacin, Tetracycline, Imipenem and Irgasan were included as positive controls for inhibition of RNA, DNA, protein, cell wall and lipid synthesis, respectively. (Figure 1) <u>DNA, RNA, and protein synthesis:</u> When cells reached early exponential phase, 100 μL of culture was added to triplicate wells containing various concentrations of MDN-0057 or control antibiotics (2.5 μL) at 40X the final concentration in 100% DMSO or deionized water ($dH_2O$). A 2.5% DMSO or $dH_2O$ alone treated culture served as the "no drug" control for all experiments. Cells were added in MHB at 102.5% to account for the volume of drug added to each reaction or in M9 minimal medium for protein synthesis reactions. Following a 5 min incubation at room temperature, either [$^3$H] thymidine (DNA synthesis), [$^3$H] uridine (RNA synthesis) or [$^3$H] leucine (protein synthesis) was added at 0.5-1.0 μCi per reaction, depending on the experiment. Reactions were allowed to proceed at room temperature for 15-40 min and then stopped by adding 12 μL of cold 5% trichloroacetic acid (TCA) or 5% TCA/2% casamino acids (protein synthesis). Reactions were incubated on ice for 30 min and the TCA precipitated material was collected on a 25 mm GF/1.2 μm PES 96 well filter plate (Corning). After washing five times with 200 μL per well of cold 5% TCA, the filters were allowed to dry, and then counted using a Packard Top Count microplate scintillation counter.

<u>Cell wall synthesis:</u> Bacterial cells in early exponential growth phase were transferred to M9 minimal medium and added to 1.5 ml eppendorf tubes (100 μl/tube) containing various concentrations of MDN-0057 or control antibiotics (2.5 μl) at 40X the final concentration in 100% DMSO or $dH_2O$ as described above. Following a 5 min incubation at 37°C, [14C] N-acetylglucosamine (0.4 μCi/reaction) was added to each tube and incubated for 30 min in a 37°C heating block. Reactions were stopped through the addition of 100 μL of 8% SDS to each tube. Reactions were then heated at 95°C for 30 min in a heating block, cooled, briefly centrifuged, and spotted onto pre-wet HA filters (0.45 μM). After washing three times with 5 ml of 0.1% SDS, the filters were rinsed two times with 5 mL of $dH_2O$, allowed to dry, and then counted using a Beckman LS3801 liquid scintillation counter.

<u>Lipid synthesis:</u> Bacterial cells were grown to early exponential growth phase in MHB and 100μL was added to 1.5 mL eppendorf tubes (in triplicate) containing various concentrations of MDN-0057 or control antibiotics as described above. Following a 5 min incubation at room temperature, [3H] glycerol was added at 0.5 μCi per reaction. Reactions were allowed to proceed at room temperature for 40 min and then stopped through the addition of 375 μL chloroform/methanol (1:2) followed by vortexing for 20 seconds after each addition. Chloroform (125μL) was then added to each reaction with vortexed, followed by the addition of 125 μL $dH_2O$ and vortexing. Reactions were centrifuged at 13,000 rpm for 10 min, and then 150 μL of the organic phase was transferred to a scintillation vial and allowed to dry in a fume hood for at least 1 hr. Samples were then counted via liquid scintillation counting.

The effect of MDN-0057 on macromolecular synthesis in E. *coli acrAB*::Tn903 is shown in figure 1. Little or no inhibition occurred for RNA, DNA, protein, or lipid synthesis when exposing E. *coli acrAB*::Tn903 cells at up to 8-fold the MIC for MDN-0057. There was an apparent dose-response inhibition of cell wall synthesis, with maximum inhibition of ca. 75% inhibition occurring at 2 to 8-fold the MIC.

## Claims

**1.** A compound of general formula (I) or a pharmaceutically acceptable salt, isomer, prodrug or solvate thereof

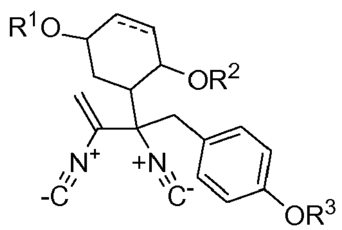

(I)

wherein each $R^1$, $R^2$ and $R^3$ are independently selected from hydrogen and a hydroxyl protecting group; and represents a single or double bond.

2. Compound according to claim 1, wherein each $R^1$, $R^2$ and $R^3$ are independently H or a hydroxyl protecting group selected from:

- ethers of formula -R';
- esters of formula -C(=O)R'; and
- carbonates of formula -C(=O)OR';

wherein R' can be independently selected from substituted or unsubstituted alkyl and substituted or unsubstituted aryl.

3. Compound according to any one of claims 1 or 2, wherein the hydroxy protecting group is independently selected from methyl ether, tert-butyl ether, benzyl ether, p-methoxybenzyl ether, 3,4-dimethoxybenzyl ether, trityl ether, allyl ether, methoxymethyl ether, 2-methoxyethoxymethyl ether, benzyloxymethyl ether, p-methoxybenzyloxymethyl ether, 2-(trimethylsilyl)ethoxymethyl, 1-methoxyethyl ether, 1-ethoxyethyl ether, 1-n-propoxyethyl ether, 1-isopropoxyethyl ether, 1-n-butoxyethyl ether, 1-isobutoxyethyl ether, 1-sec-butoxyethyl ether, 1-*tert*-butoxyethyl ether, 1-ethoxy-n-propyl ether, methoxypropyl ether, ethoxypropyl ether, 1-methoxy-1-methylethyl ether, 1-ethoxy-1-methylethyl ether; tetrahydropyranyl; acetate, hydroxyacetate, benzoate, pivaloate, methoxyacetate, chloroacetate, levulinate; benzyl carbonate, p-nitrobenzyl carbonate, tert-butyl carbonate, 2,2,2-trichloroethyl carbonate, 2-(trimethylsilyl)ethyl carbonate, allyl carbonate.

4. Compound according to any one of claims 1 to 3, wherein $R^1$ is H.

5. Compound according to any one of claims 1 to 4, wherein $R^2$ is H, $-COCH_3$ or $-COCH_2OH$.

6. Compound according to any one of claims 1 to 5, wherein $R^3$ is H or $C_1$-$C_{18}$ alkyl.

7. Compound according to claim 6, wherein $R^3$ is $C_1$-$C_4$ alkyl.

8. Compound according to claim 7, wherein $R^3$ is methyl.

9. Compound according to any one of claims 1 to 8, which is selected from the group consisting of:

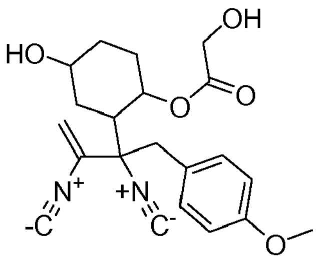,
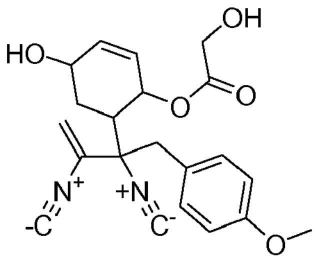,

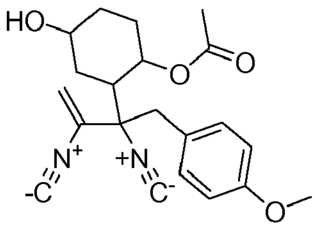 and
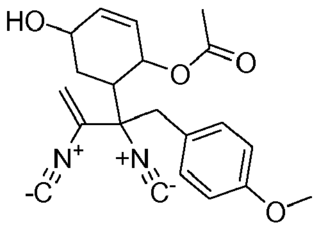,

or a pharmaceutically acceptable salt, isomer, prodrug or solvate thereof.

10. Compound according to claim 9, which is selected from the group consisting of:

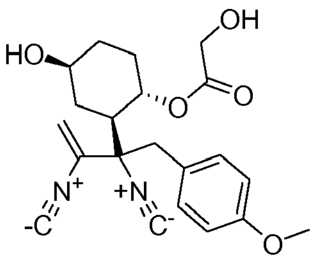,

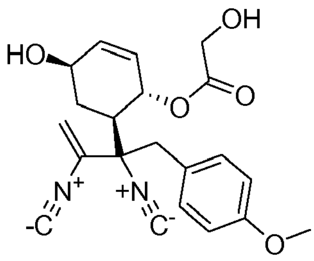,

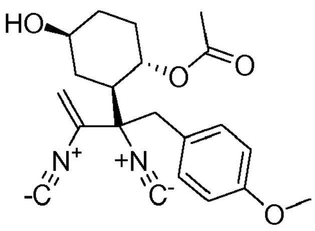 and 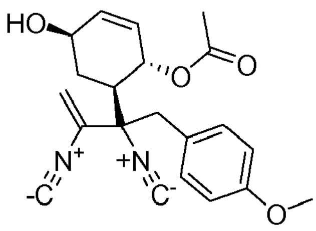,

or a pharmaceutically acceptable salt, prodrug or solvate thereof.

**11.** Pharmaceutical composition comprising at least one compound of formula (I) as defined in any one of claims 1 to 10, or a pharmaceutically acceptable salt, isomer, prodrug or solvate thereof and a pharmaceutically acceptable excipient.

**12.** Compound according to any one of claims 1 to 10 for use in medicine.

**13.** Compound according to any one of claims 1 to 10 for use in the treatment and/or prophylaxis of a bacterial infection disease.

**14.** Compound according to any one of claims 1 to 10 for a use as defined in claim 13, wherein the bacterial infection disease is caused by a gram-negative bacteria.

**15.** A process for obtaining a compound of general formula (I) as defined in any one of claims 1 to 10 or a pharmaceutically acceptable salt, isomer, prodrug or solvate thereof, said process comprising the steps of cultivating the strain of O. *korrae* CBS 102216 in an aqueous nutrient medium with assimilable carbon and nitrogen sources and salts, under controlled submerged aerobic conditions, and then recovering and purifying the compound of general formula (I) from the cultured broth.

Figure 1

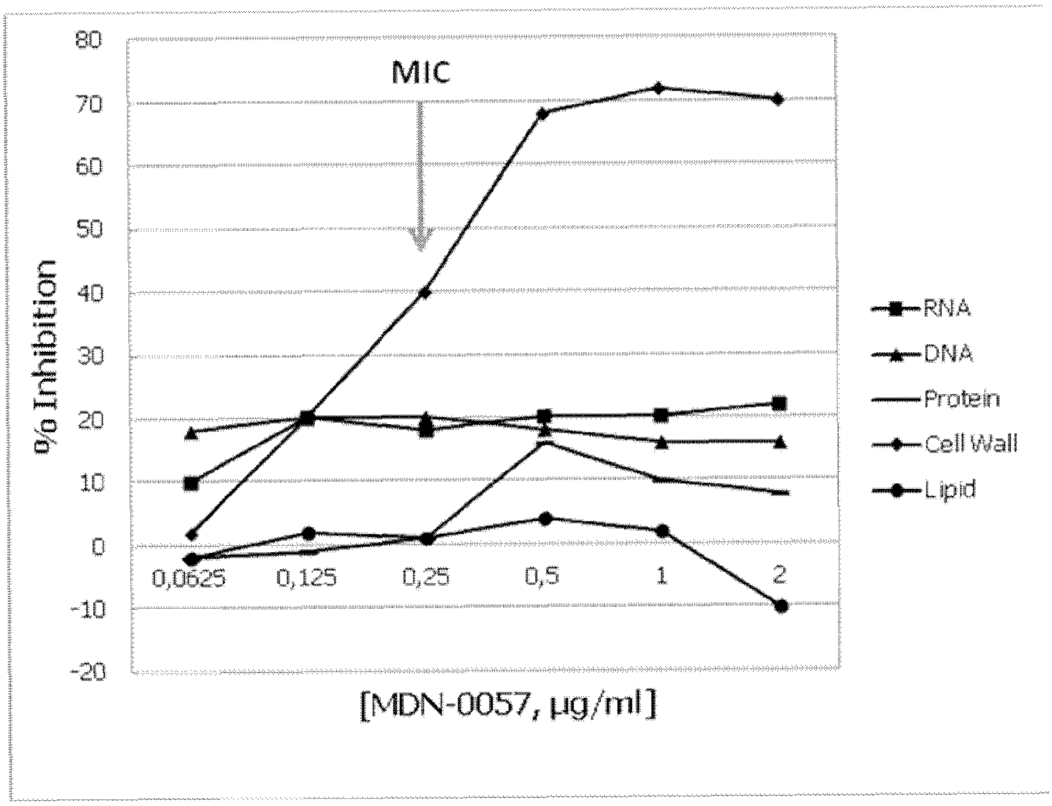

MIC
% Inhibition
80
70
60
50
40
30
20
10
0
-10
-20
0,0625
0,125
0,25
0,5
1
2
[MDN-0057, µg/ml]
RNA
DNA
Protein
Cell Wall
Lipid

Europäisches Patentamt
European Patent Office
Office européen des brevets

# EUROPEAN SEARCH REPORT

Application Number
EP 13 38 2258

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 537 777 A (ANDERSON MARIE T [US] ET AL) 27 August 1985 (1985-08-27)<br>* column 9, line 8 - line 40; claims 1-9; table IX *<br>----- | 1-15 | INV.<br>C07C291/10<br>A61P1/00<br>A61K31/277 |
| A | YING ZHAO ET AL: "SD118-Xanthocillin X (1), a Novel Marine Agent Extracted from Penicillium commune, Induces Autophagy through the Inhibition of the MEK/ERK Pathway",<br>MARINE DRUGS,<br>vol. 10, no. 12,<br>11 December 2012 (2012-12-11), pages 1345-1359, XP55086422,<br>ISSN: 1660-3397, DOI: 10.3390/md10061345<br>* figure 1 *<br>----- | 1-15 | |
| A | E K S VLJAYAKUMAR ET AL: "L 970843 and L 970844, Two New Antifungal Metabolites from an Unidentified Fungal Species HIL Y-903146f",<br>THE JOURNAL OF ANTIBIOTICS,<br>1 January 2001 (2001-01-01), XP55086437,<br>* figure 1; table 2 *<br>----- | 1-15 | TECHNICAL FIELDS SEARCHED (IPC)<br>C07C |

The present search report has been drawn up for all claims

1

| Place of search | Date of completion of the search | Examiner |
|---|---|---|
| The Hague | 1 November 2013 | Scheid, Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 38 2258

This annex lists the patent family members relating to the patent documents cited in the above-mentioned European search report.
The members are as contained in the European Patent Office EDP file on
The European Patent Office is in no way liable for these particulars which are merely given for the purpose of information.

01-11-2013

| Patent document cited in search report | Publication date | Patent family member(s) | Publication date |
|---|---|---|---|
| US 4537777 A | 27-08-1985 | DK 589584 A | 17-06-1985 |
| | | EP 0146346 A2 | 26-06-1985 |
| | | GB 2153817 A | 29-08-1985 |
| | | GR 81212 A1 | 08-04-1985 |
| | | JP S60241891 A | 30-11-1985 |
| | | US 4537777 A | 27-08-1985 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **F. D. LOWY.** *The Journal of Clinical Investigation,* 2003, vol. 111 (9), 1265
- **GEORGE TALBOT et al.** Bad Bugs Need Drugs: An Update on the Development Pipeline from the Antimicrobial Availability Task Force of the Infectious Disease Society of America. *CLINICAL INFECTIOUS DISEASES,* 2006, vol. 42, 657
- **BRAD SPELLBERG et al.** The Epidemic of Antibiotic-Resistant Infections: A Call to Action for the Medical Community from the Infectious Disease Society of America. *CLINICAL INFECTIOUS DISEASES,* 2007, vol. 46, 155
- **HELEN W. BOUCHER et al.** 10 x '20 Progress-Development of New Drugs Active Against Gram-Negative Bacilli: An Update From the Infectious Diseases Society of America. *Clin Infect Dis,* 2013
- **WUTS, PGM ; GREENE TW.** Protecting Groups in Organic Synthesis. Wiley-Interscience
- **KOCIENSKI PJ.** Protecting Groups. Georg Thieme Verlag
- **BURGER.** Medicinal Chemistry and Drug Discovery. Wiley, 2001
- Design and Applications of Prodrugs. Harwood Academic Publishers, 1985
- **KROGSGAARD-LARSEN et al.** Textbook of Drug design and Discovery. Taylor & Francis, April 2002
- March's Advanced Organic Chemistry. Wiley Interscience, 2007
- U.S. National Library of Medicine. National Institutes of Health
- Stedman's Medical Spellchecker. Lippincott Williams & Wilkins, 2006
- **G. BILLS ; G. PLATAS ; A. FILLOLA ; M.R. JIMÉNEZ ; J. COLLADO ; F. VICENTE ; J. MARTÍN ; A. GONZÁLEZ ; J. BUR-ZIMMERMANN ; J.R. TORMO.** Enhancement of antibiotic and secondary metabolite detection from filamentous fungi by growth on nutritional arrays. *Journal of Applied Microbiology,* 2008, vol. 104, 1644-1658
- Methods for Dilution Antimicrobial Susceptibility Tests for Bacteria that Grow Aerobically; Approved Standard. Clinical Laboratory Standards Institute publication M07-A9. January 2012, vol. 32